# EUROPÄISCHE PATENTSCHRIFT

(11) **EP 1 682 468 B1**
(45) Veröffentlichungstag und Bekanntmachung des Hinweises auf die Patenterteilung: **15.07.2009**
(21) Anmeldenummer: 04790913.0
(22) Anmeldetag: 27.10.2004
(51) Int. Cl.: C07C 5/333, C07C 5/25, C07C 5/05, C07C 7/08

(54) **VERFAHREN ZUR HERSTELLUNG VON 1-BUTEN**
METHOD FOR PRODUCING 1-BUTENE
PROCEDE DE FABRICATION DE 1-BUTENE

(30) Priorität: 27.10.2003 DE 10350044
(43) Veröffentlichungstag der Anmeldung: 26.07.2006
(73) Patentinhaber: BASF SE, 67056 Ludwigshafen (DE)
(72) Erfinder: SCHINDLER, Götz-Peter, 68219 Mannheim (DE); BRODHAGEN, Andreas, 67125 Dannstadt-Schauernheim (DE); JOHANN, Thorsten, 67117 Limburgerhof (DE); HILL, Thomas, 67071 Ludwigshafen (DE); SIGL, Marcus, 68167 Mannheim (DE); BENFER, Regina, 67122 Altrip (DE)
(74) Vertreter: Huhn, Michael
(86) Internationale Anmeldenummer: PCT/EP2004/012138
(87) Internationale Veröffentlichungsnummer: WO 2005/042449

(56) Entgegenhaltungen:
- EP-A- 0 129 900
- EP-A- 0 751 106
- WO-A-20/04007408
- US-A- 4 558 168
- US-A- 4 718 986
- US-A- 5 087 780

## Beschreibung

Die Erfindung betrifft ein Verfahren zur Herstellung von 1-Buten.

Butene können durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butenen, Butadien, Butinen, Methylallen, C₅- und höheren Kohlenwasserstoffen an.

Nachteilig an der Erzeugung von Buten im Crackprozess ist, dass zwangsläufig größere Mengen an Ethen oder Propen als Koppelprodukte anfallen.

Alternativ können Butene ausgehend von n-Butan durch katalytische Dehydrierung hergestellt werden. Nachteilig an diesen Verfahren ist jedoch, dass bei der katalytischen Dehydrierung von n-Butan neben 1-Buten auch 2-Buten und Butadien in größeren Mengen entsteht.

Aufgabe der Erfindung ist es, ein Verfahren zur Herstellung von 1-Buten aus n-Butan bereitzustellen, bei dem in möglichst geringem Umfang Koppelprodukte anfallen.

Verfahren zur Herstellung von I-Buten werden in US-4,558,168 und EP- 0 751106 offenbart.

Gelöst wird die Aufgabe durch ein Verfahren zur Herstellung von 1-Buten aus n-Butan mit den Schritten
A) Bereitstellung eines n-Butan enthaltenden Einsatzgasstroms a;
B) Einspeisung des n-Butan enthaltenden Einsatzgasstroms a in mindestens eine Dehydrierzone und nicht-oxidative katalytische Dehydrierung von n-Butan, wobei ein Produktgasstrom b enthaltend n-Butan, 1-Buten, 2-Buten, Butadien, leichtsiedende Nebenbestandteile, Wasserstoff und gegebenenfalls Wasserdampf erhalten wird;
C) Abtrennung von Wasserstoff, der leichtsiedenden Nebenbestandteile und gegebenenfalls von Wasserdampf, wobei ein C₄-Produktgasstrom c im wesentlichen bestehend aus n-Butan, 1-Buten, 2-Buten und Butadien erhalten wird;
D) Trennung des C₄-Produktgasstroms c in einen im Wesentlichen aus n-Butan bestehenden Rückführstrom d1 und einen 1-Buten, 2-Buten und Butadien enthaltenden Strom d2 durch Extraktivdestillation und Rückführung des im Wesentlichen aus n-Butan bestehenden Rückführstroms d1 in die Dehydrierzone;
E) Einspeisung des 1-Buten, 2-Buten und Butadien enthaltenden Stroms d2 in eine Selektivhydrierungszone und Selektivhydrierung von Butadien zu 1-Buten und/oder 2-Buten, wobei ein 1-Buten und 2-Buten enthaltender Strom e erhalten wird;
F) Einspeisung des 1-Buten und 2-Buten enthaltenden Stroms e und eines 1-Buten und 2-Buten enthaltenden Kreisstroms g in eine Destillationszone und Abtrennung eines im Wesentlichen aus 1-Buten bestehenden Wertproduktstroms f1, wobei ein 2-Buten enthaltender Strom f2 verbleibt;
G) Einspeisung des 2-Buten enthaltenden Stroms f2, gegebenenfalls nach Abtrennung eines Purgegasstroms, in eine Isomerisierungszone und Isomerisierung von 2-Buten zu 1-Buten, wobei ein 1-Buten und 2-Buten enthaltender Kreisstrom g gewonnen wird, und Rückführung, gegebenenfalls nach Abtrennung eines Purgegasstroms aus dem Kreisgasstrom g, des Kreisgasstroms g in die Destillationszone.

Das erfindungsgemäße Verfahren zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. N-Butan wird auf diese Weise praktisch vollständig umgesetzt. Bei der Dehydrierung gebildetes Butadien wird durch Selektivhydrierung in weiteres Wertprodukt umgewandelt, bei der Deyhdrierung bzw. der Selektivhydrierung gebildetes 2-Buten durch Isomerisierung in Wertprodukt umgewandelt. Es fallen somit weder Butadien noch 2-Buten als Koppelprodukte an. Ferner wird wegen der höheren Selektivität der nicht-oxidativen Butan-Dehydrierung gegenüber dem Steam-Cracken der Anfall von Ethen und Propen minimiert.

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im wesentlichen C₂-C₅-Kohlenwasserstoffe. Daneben enthält es auch Methan und Spuren von C₆⁺-Kohlenwasserstoffen. Die Zusammensetzung von LPG kann stark schwanken. Vorteilhafter Weise enthält das eingesetzte LPG mindestens 10 Gew.-% Butane.

Alternativ kann ein veredelter C₄-Strom aus Crackern oder Raffinerien eingesetzt werden.

In einer Variante des erfindungsgemäßen Verfahrens umfasst die Bereitstellung des n-Butan enthaltenden Dehydrier-Einsatzgasstromes die Schritte
(A1) Bereitstellung eines liquefied petroleum gas (LPG)-Stroms,
(A2) Abtrennung von Propan und gegebenenfalls Methan, Ethan und C₅⁺-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom wird vorzugsweise einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether eingesetzt werden.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden.

Ein Merkmal der nicht-oxidativen Fahrweise gegenüber einer oxidativen Fahrweise ist das Vorhandensein von Wasserstoff im Austragsgas. Bei der oxidativen Dehydrierung wird kein freier Wasserstoff in wesentlichen Mengen gebildet.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff und/oder Kohlenwasserstoffen in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne Sauerstoffhaltigem Gas als Co-Feed in einem Hordenreaktor durchgeführt werden. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stellen des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100°C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweise, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III, und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispiel 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaltigen Gas reduziert.

Bei der n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 1 bis 15 Vol.-% 1-Buten, 1 bis 25 Vol.-% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 70 Vol.-% Stickstoff und 0 bis 5 Vol.-% Kohlenstoffoxide.

In einem Verfahrensteil C werden die von den C₄-Kohlenwasserstoffen (n-Butan, iso-Butan, 1-Buten, cis-/trans-2-Buten, iso-Buten, Butadien) verschiedenen leicht siedenden Nebenbestandteile zumindest teilweise, vorzugsweise aber im wesentlichen vollständig aus dem Produktgasstrom der n-Butan-Dehydrierung abgetrennt, wobei ein C₄-Produktgasstrom c erhalten wird.

Der die Dehydrierzone verlassende Produktgasstrom b wird bevorzugt in zwei Teilströme aufgetrennt, wobei nur einer der beiden Teilströme den weiteren Verfahrensteilen C bis G unterworfen wird und der zweite Teilstrom in die Dehydrierzone zurückgeführt werden kann. Eine entsprechende Verfahrensweise ist in DE-A 102 11 275 beschrieben. Es kann jedoch auch der gesamte Produktgasstrom b der n-Butan-Dehydrierung den weiteren Verfahrensteilen C bis G unterworfen werden.

In einer Ausführungsform des erfindungsgemäßen Verfahrens wird im Verfahrensteil C zunächst Wasser aus dem Produktgasstrom b abgetrennt. Die Abtrennung von Wasser kann beispielsweise durch Auskondensieren durch Abkühlen und/oder Verdichten des Produktgasstroms b erfolgen und kann in einer oder mehreren Abkühlungs- und/oder Verdichtungsstufen durchgeführt werden. Die Wasserabtrennung wird üblicherweise durchgeführt, wenn die n-Butan-Dehydrierung autotherm durchgeführt oder unter Einspeisung von Wasserdampf isotherm (analog dem Linde- oder STAR-Prozess zur Dehydrierung von Propan) durchgeführt wird und folglich der Produktgasstrom b einen hohen Wasseranteil aufweist.

Die Abtrennung der leicht siedenden Nebenbestandteile aus dem Produktgasstrom kann durch übliche Trennverfahren wie Destillation, Rektifikation, Membranverfahren, Absorption oder Adsorption erfolgen.

Zur Abtrennung des im Produktgasstrom b der n-Butan-Dehydrierung enthaltenen Wasserstoffs kann das Produktgasgemisch, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

Das in dem Produktgasstrom b der Dehydrierung enthaltene Kohlendioxid kann durch CO₂-Gaswäsch abgetrennt werden. Der Kohlendioxid-Gaswäsche kann eine gesonderte Verbrennungsstufe vorgeschaltet werden, in der Kohlenmonoxid selektiv zu Kohlendioxid oxidiert wird.

In einer bevorzugten Ausführungsform des erfindungsgemäßen Verfahrens werden die nicht kondensierbaren oder leicht siedenden Gasbestandteile wie Wasserstoff, Kohlenstoffoxide, die leicht siedenden Kohlenwasserstoffe (Methan, Ethan, Ethen, Propan, Propen) und gegebenenfalls Stickstoff in einem Absorptions-/Desorptions-Cyclus mittels eines hoch siedenden Absorptionsmittels abgetrennt, wobei ein C₄-Produktgasstrom c erhalten wird, der im Wesentlichen aus den C₄-Kohlenwasserstoffen besteht. Im Allgemeinen besteht der C₄-Produktgasstrom c zu mindestens 80 Vol.-%, bevorzugt zu mindestens 90 Vol.-%, besonders bevorzugt zu mindestens 95 Vol.-% aus den C₄-Kohlenwasserstoffen.

Dazu wird in einer Absorptionsstufe der Produktgasstrom b - gegebenenfalls nach vorheriger Wasserabtrennung - mit einem inerten Absorptionsmittel in Kontakt gebracht und werden die C₄-Kohlenwasserstoffe in dem inerten Absorptionsmittel absorbiert, wobei mit C₄-Kohlenwasserstoffen beladenes Absorptionsmittel und ein die übrigen Gasbestandteile enthaltendes Abgas erhalten werden. In einer Desorptionsstufe werden die C₄-Kohlenwasserstoffe aus dem Absorptionsmittel wieder freigesetzt.

In der Absorptionsstufe eingesetzte inerte Absorptionsmittel sind im allgemeinen hochsiedende unpolare Lösungsmittel, in denen das abzutrennende C₄-Kohlenwasserstoff-Gemisch eine deutlich höhere Löslichkeit als die übrigen, abzutrennenden Gasbestandteile aufweist. Die Absorption kann durch einfaches Durchleiten des Produktgasstroms b durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption der C₄-Kohlenwasserstoffe wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

In einer Verfahrensvariante wird der Desorptionsschritt durch Entspannung und/oder Erhitzen des beladenen Desorptionsmittels durchgeführt.

Die Abtrennung C ist im Allgemeinen nicht ganz vollständig, so dass in dem C₄-Produktgasstrom - je nach Art der Abtrennung - noch geringe Mengen oder auch nur Spuren der weiteren Gasbestandteile, insbesondere der leicht siedenden Kohlenwasserstoffe, vorliegen können.

Die durch die Abtrennung C auch bewirkte Volumenstromverringerung entlastet die nachfolgenden Verfahrensschritte.

Der im Wesentlichen aus n-Butan, 1-Buten, 2-Buten und Butadien bestehende C₄-Produktgasstrom c enthält im Allgemeinen 20 bis 80 Vol.-% n-Butan, 5 bis 40 Vol.-% 1-Buten, 10 bis 50 Von.-% 2-Buten und 0 bis 30 Vol.-% Butadien.

In einem Verfahrensteil D wird der C₄-Produktgasstrom c mittels Extraktivdestillation in einen im wesentlichen aus n-Butan bestehenden Rückführstrom d1 und einen 1-Buten, 2-Buten, Butadien und n-Butan enthaltenden Strom d2 aufgetrennt. Hierzu wird der C₄-Produktgasstrom c mit einem Extraktionsmittel, vorzugsweise ein N-Methylpyrrolidon (NMP)/Wasser-Gemisch, in einer Extraktionszone in Kontakt gebracht, wobei die Butene und Butadien im Wesentlichen vollständig absorbiert werden, die Butane (n-Butan und iso-Butan) jedoch im Wesentlichen in der Gasphase verbleiben. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Böden auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen NMP mittels flüssigen Kohlenwasserstoffrücklaufs, wozu die Kopffraktion zuvor kondensiert wird. Typische Temperaturen am Kopf der Kolonne liegen zwischen 30 und 60°C. Das Massenverhältnis NMP zu C₄-Produktgasstrom c im Zulauf der Extraktionszone beträgt im allgemeinen 10 : 1 bis 20 : 1.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte cyclische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmittel und/oder tert.-Butylether, z. B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

In der Extraktionszone wird ein gasförmiger Butanstrom d1 und eine Butene und Butadien enthaltende Extraktionslösung gebildet. Der gasförmige Butanstrom d1 wird als Rückführstrom d1 in die n-Butan-Dehydrierzone zurückgeführt. Im Allgemeinen enthält der im Wesentlichen aus n-Butan bestehende Rückführstrom d1 82 bis 99 Vol.-% n-Butan, 1 bis 15 Vol.-% Butene und 0 bis 3 Vol.-% weitere Bestandteile wie Butadien und iso-Butan.

Die Extraktionslösung wird in eine Desorptionszone mit gegenüber der Extraktionszone vermindertem Druck und/oder erhöhter Temperatur überführt, wobei aus der Extraktionslösung die Butene und Butadien desorbiert werden. Die Desorptionszone kann beispielsweise in Form einer Waschkolonne ausgeführt sein, die 5 bis 15, bevorzugt 8 bis 10 theoretische Stufen und eine Rückwaschzone mit beispielsweise 4 theoretischen Stufen aufweist. Diese Rückwaschzone dient zur Rückgewinnung des in der Gasphase enthaltenen NMP mittels flüssigem Kohlenwasserstoffrücklaufes, wozu die Kopffraktion zuvor kondensiert wird. Als Einbauten sind Packungen, Böden oder Füllkörper vorgesehen. Der Druck am Kolonnenkopf beträgt beispielsweise 1,5 bar. Die Temperatur im Kolonnensumpf liegt beispielsweise bei 130 bis 150°C.

Der am Kolonnenkopf gewonnene Strom d2 enthält im Allgemeinen 0 bis 10 Vol.-% n-Butan, 20 bis 60 Vol.-% 1-Buten, 40 bis 80 Vol.-% 2-Butene und 0 bis 50 Vol.-% Butadien:
In einem Verfahrensteil E wird der überwiegend 1-Buten, 2-Buten und Butadien enthaltende Strom d2 in eine Selektivhydrierungszone eingespeist und wird eine Selektivhydrierung von Butadien zu 1-Buten und/oder 2-Buten durchgeführt.

Die Selektivhydrierung kann in an sich bekannter Weise in der Gasphase, Flüssigphase oder Rieselphase durchgeführt werden. Vorzugsweise wird die Selektivhydrierung in der Flüssig- oder Rieselphase mit fest angeordnetem Hydrierkatalysator durchgeführt. Als Hydrierkatalysatoren können Edelmetall-Trägerkatalysatoren eingesetzt werden, die Palladium, Platin, Silber, Gold, Rhodium, Ruthenium, Osmium oder Gemische dieser Metalle enthalten. Als Hydrierkatalysatoren sind Palladium enthaltende Trägerkatalysatoren besonders bevorzugt. Ein bevorzugt eingesetzter Hydrierkatalysator ist beispielsweise in EP-A 0 992 284 beschrieben. Dieser enthält Metalle der 8., 9. und 10. Gruppe des Periodensystems, insbesondere Ruthenium, Rhodium, Palladium und/oder Platin, auf einem Aluminiumoxid-Träger und daneben noch mindestens ein Metall der 11. Gruppe des Periodensystems, bevorzugt Kupfer und/oder Silber. Der Gehalt des Katalysators an Metall der 8., 9. oder 10. Gruppe des Periodensystems beträgt dabei im Allgemeinen 0,05 bis 2 Gew.-%, bevorzugt 0,1 bis 0,5 Gew.-%.

Die Selektivhydrierung kann in einem oder mehreren hintereinander geschalteten Reaktoren durchgeführt werden. Die Selektivhydrierung kann beispielsweise zweistufig ausgeführt werden. Die Temperatur liegt üblicher Weise im Bereich von 0°C bis 180°C und der Druck im Bereich von 2 bis 50 bar. In einer Ausführungsform wird die Selektivhydrierung bei einer Temperatur von 20 bis 90°C und einem Druck im Bereich von 5 bis 50 bar durchgeführt, wobei je Mol Butadien 1 bis 1,5 Mol Wasserstoff zugegeben wird.

Der die Selektivhydrierungszone verlassende Strom e enthält überwiegend 1-Buten und 2-Buten und daneben n-Butan. Im Allgemeinen enthält der Strom e 20 bis 50 Vol.-% 1-Buten, 50 bis 80 Vol.-% 2-Buten und 0 bis 10 Vol.-% n-Butan. Daneben kann der Strom e noch in geringen Mengen weitere Gasbestandteile enthalten wie Isobutan, Isobuten und Butadien, im Allgemeinen in Mengen von 0 bis 5 Vol.-%, bevorzugt 0 bis 1 Vol.-%.

In einem Verfahrensteil F wird der 1-Buten und 2-Buten enthaltende Strome und ein 1-Buten und 2-Buten enthaltenden Kreisstroms g, der in einem stromabwärts gelegenen Verfahrensteil G durch Isomerisierung von 2-Buten gewonnen wird, in eine Destillationszone eingespeist. Dort wird ein im Wesentlichen aus 1-Buten bestehenden Wertproduktstroms f1 gewonnen, wobei ein 2-Buten enthaltender Strom f2 verbleibt.

Die Destillationszone besteht im Allgemeinen aus einer Destillationskofonne mit im Allgemeinen 30 bis 80, bevorzugt 40 bis 75 theoretischen Trennstufen. Geeignet sind beispielsweise Glockenbodenkolonnen, Füllkörperkolonnen, Packungskolonnen oder Trennwandkolonnen. Das Rücklaufverhältnis beträgt im Allgemeinen 10 bis 50. Die Destillation wird im Allgemeinen bei einem Druck von 5 bis 20 bar durchgeführt.

Im oberen Teil der Kolonne, vorzugsweise am Kolonnenkopf wird ein im Wesentlichen aus 1-Buten bestehender Strom f1 abgezogen. Dieser enthält im Allgemeinen mindestens 90 Vol.-%, vorzugsweise mindestens 95 Vol.-% 1-Buten und daneben bis zu 10 Vol.-%, vorzugsweise bis zu 5 Vol.-% weitere Bestandteile wie n-Butan, Isobutan, Iso-buten, Butadien und 2-Buten.

Im unteren Teil der Kolonne, vorzugsweise im unteren Fünftel der Kolonne, besonders bevorzugt am Kolonnensumpf bis maximal 5 theoretische Böden oberhalb des Kolonnensumpfs, wird ein 2-Buten enthaltender Strom f2 abgezogen. Üblicherweise enthält der Strom f2 55 bis 95 Vol.-% 2-Buten und daneben 0 bis 30 Vol.-% n-Butan und 0 bis 15 Vol.-% 1-Buten. Von dem 2-Buten enthaltenden Strom f2 kann ein Purgegasstrom abgetrennt werden, um die Aufpegelung von Hochsiedern zu vermeiden. Der Purgegasstrom kann in die Dehydrierzone zurückgeführt werden.

In einem Verfahrensteil G wird der 2-Buten enthaltende Strom f2 in eine Isomerisierungszone eingespeist und eine Isomerisierung von 2-Buten zu 1-Buten durchgeführt. Dabei wird der 2-Buten enthaltende Strom f2 über einen Isomerisierungskatalysator geleitet. Geeignete Isomerisierungskatalysatoren sind basische Katalysatoren oder Katalysatoren auf Zeolithbasis. Daneben kann die Isomerisierung auch unter hydrierenden Bedingungen an Edelmetall-haltigen Katalysatoren erfolgen.

Als Katalysatoren eignen sich Erdalkalioxide auf Aluminiumoxid, wie in EP-A 0 718 036 beschrieben, gemischte Aluminiumoxid/Siliziumoxid-Träger, die mit Oxiden der Erdalkalimetalle, Borgruppenmetalle, Lanthaniden oder Elementen der Eisengruppe dotiert sind, wie in US 4,814,542 beschrieben, oder mit Alkalimetallen belegtes gamma-Aluminiumoxid, wie in JP 51/108691 beschrieben. Weiterhin eignen sich Manganoxid auf Aluminiumoxid, wie in US 4,289,919 beschrieben, Magnesium-, Alkalimetall- und Zirkonoxid dispergiert auf einem Aluminiumoxid-Träger, wie in EP-A 0 234 498 beschrieben, sowie Aluminiumoxidkatalysatoren, die zusätzlich Natriumoxid und Siliziumdioxid enthalten, wie in US 4,229,610 beschrieben.

Geeignete Zeolith-basierte Katalysatoren sind beschrieben in EP-A 0 129 899. Geeignet sind weiterhin mit Alkali- oder Erdalkalimetallen ausgetauschte Molsiebe, wie in US 3,475,511 beschrieben, Alumosilikate, wie in US 4,749,819 beschrieben, sowie Zeolithe in Alkali- oder Erdalkaliform, wie in US 4,992,613 beschrieben, und solche auf Basis kristalliner Borosilikate, wie in US 4,499,326 beschrieben.

Die Katalysatoren werden üblicher Weise im Festbett, Wirbelbett oder Wanderbett eingesetzt. Für die Isomerisierung ist ein kontinuierlich durchströmter Festbett-Reaktor bevorzugt. Geeignete Reaktoren sind Rohrreaktoren, Rohrbündelreaktoren, Wickelreaktoren oder Wendelreaktoren. Die Isomerisierungstemperatur liegt im Aligemeinen bei 100 bis 700°C, bevorzugt bei 200 bis 500°C. Der Druck beträgt im allgemeinen 1 bis 30 bar, bevorzugt 3 bis 20 bar.

Es wird ein Kreisgasstrom g erhalten, dessen Gehalt an 2-Buten um 5 bis 30 Vol.-% niedriger ist als im Strom f2. Im Allgemeinen enthält der Strom f2 8 bis 25 Vol.-% 1-Buten, 45 bis 90 Vol.-% 2-Buten und 0 bis 30 Vol.-% n-Butan. Der Kreisgasstrom g wird zusammen mit dem 1-Buten und 2-Buten enthaltenden Strom e in die Destillationszone eingespeist. Von dem Kreisgasstrom g kann ein Purgegasstrom abgetrennt werden. Dieser kann in die Dehydrierzone zurückgeführt werden.

### Beispiel

Ein Einsatzgasstrom a aus 22 857 kg/h n-Butan wird zusammen mit 1 965 kg/h Sauerstoff und 14 735 kg/h Wasserdampf in einen Dehydrierreaktor eingespeist und bei 650°C einer autothermen, nicht-oxidativen katalytischen Dehydrierung unterzogen.

Es wird ein Produktgasstrom b der nachstehenden Zusammensetzung erhalten (alle nachstehenden %-Angaben sind Massen-%): n-Butan 39,9%, iso-Butan 0,3%, 1-Buten 9,3%, cis-2-Buten 7,1%, trans-2-Buten 9,6%, iso-Buten 0,3%, Butadien 4,8%, Propan 0,1%, Propen 0,7%, Wasserdampf 24,4%, CO₂ 1,5%, CO 0,3%, Sauerstoff 0,2%, Wasserstoff 1,1%, Ethan 0,2%, Ethen 0,1%, Methan 0,1%.

Aus dem Produktgasstrom b wird Wasserdampf (15 937 kg/h) auskondensiert, anschließend werden leicht siedende Nebenkomponenten abgetrennt (CO₂ 953 kg/h, CO 178 kg/h, O₂ 101 kg/h, H₂ 705 kg/h, Ethan 114 kg/h, Ethen 84 kg/h, Methan 66 kg/h, Propan 90 kg/h, Propen 443 kg/h).

Der verbleibende C₄-Produktgasstrom c wird anschließend in einer Extraktivdestillation in einen im wesentlichen aus n-Butan bestehenden Rückführstrom d1 (26 125 kg/h), der in den Dehydrierreaktor zurückgeführt wird, und einen im wesentlichen aus n-Butan, 1-Buten, 2-Buten und Butadien bestehenden Strom d2 (20 410 kg/h) aufgetrennt.

Die Zusammensetzung des Rückführstroms d1 ist wie folgt: n-Butan 93,5%, iso-Butan 0,7%, 1-Buten 1,7%, cis-2-Buten 1,3%, trans-2-Buten 1,8%, Butadien 0,9%.

Die Zusammensetzung des Stroms d2 ist wie folgt: n-Butan 7,8%, iso-Butan 0,1%, 1-Buten 27,6%, cis-2-Buten 21,2%, trans-2-Buten 28,4%, Isobuten 0,8%, Butadien 14,19%.

Der Strom d2 wird nachfolgend mit 108 kg/h in eine Selektivhydrierungszone eingespeist und einer Selektivhydrierung unterworfen. Es wird ein Strom e (20 518 kg/h) der nachstehenden Zusammensetzung erhalten: n-Butan 7,8%, iso-Butan 0,1%, 1-Buten 36,4%, cis-2-Buten 23,8%, trans-2-Buten 31,1%, Isobuten 0,8%, Butadien 0,01%.

In der nachfolgenden Isomerisierung/Destillation werden ein im Wesentlichen aus 1-Buten bestehender Wertproduktstrom f1 (17 018 kg/h) und ein 2-Buten enthaltender Strom f2 (3 500 kg/h) gewonnen.

Der Wertproduktstrom f1 weist die folgende Zusammensetzung auf: n-Butan 3,0%, iso-Butan 0,1 %, 1-Buten 95,9%, 2-Butene 0,1 %, Isobuten 0,9%, Butadien 0,012%.

Der Strom f2 weist die folgende Zusammensetzung auf: 31,4% n-Butan, 4,7% 1-Buten, 63,9% cis-2-Buten.

## Patentansprüche

1. Verfahren zur Herstellung von 1-Buten aus n-Butan mit den Schritten
A) Bereitstellung eines n-Butan enthaltenden Einsatzgasstroms a;
B) Einspeisung des n-Butan enthaltenden Einsatzgasstroms a in mindestens eine Dehydrierzone und nicht-oxidative, katalytische Dehydrierung von n-Butan, wobei ein Produktgasstrom b enthaltend n-Butan, 1-Buten, 2-Buten, Butadien, Wasserstoff, leichtsiedende Nebenbestandteile und gegebenenfalls Wasserdampf erhalten wird;
C) Abtrennung von Wasserstoff, der leichtsiedenden Nebenbestandteile und gegebenenfalls von Wasserdampf, wobei ein C₄-Produktgasstrom c im Wesentlichen bestehend aus n-Butan, 1-Buten, 2-Buten und Butadien erhalten wird;
D) Trennung des C₄-Produktgasstroms c in einen im Wesentlichen aus n-Butan bestehenden Rückführstrom d1 und einen 1-Buten, 2-Buten und Butadien enthaltenden Strom d2 durch Extraktivdestillation und Rückführung des im Wesentlichen aus n-Butan bestehenden Rückführstroms d1 in die Dehydrierzone;
E) Einspeisung des 1-Buten, 2-Buten und Butadien enthaltenden Stroms d2 in eine Selektivhydrierungszone und Selektivhydrierung von Butadien zu 1-Buten und/oder 2-Buten, wobei ein 1-Buten und 2-Buten enthaltender Strom e erhalten wird;
F) Einspeisung des 1-Buten und 2-Buten enthaltenden Stroms e und eines 1-Buten und 2-Buten enthaltenden Kreisstroms g in eine Destillationszone und Abtrennung eines im Wesentlichen aus 1-Buten bestehenden Wertproduktstroms f1, wobei ein 2-Buten enthaltender Strom f2 verbleibt;
G) Einspeisung des 2-Buten enthaltenden Stroms f2, gegebenenfalls nach Abtrennung eines Purgegasstroms, in eine Isomerisierungszone und Isomerisierung von 2-Buten zu 1-Buten, wobei ein 1-Buten und 2-Buten enthaltender Kreisstrom g gewonnen wird, und Rückführung, gegebenenfalls nach Abtrennung eines Purgegasstroms aus dem Kreisgasstrom g, des Kreisgasstroms g in die Destillationszone.

2. Verfahren nach Anspruch 1, **dadurch gekennzeichnet, dass** die nicht-oxidative, katalytische Dehydrierung als autotherme Dehydrierung durchgeführt wird.

3. Verfahren nach Anspruch 1 oder 2, **dadurch gekennzeichnet, dass** die Extraktivdestillation mit N-Methylpyrrolidon/Wasser-Gemisch als Extraktionsmittel durchgeführt wird.

4. Verfahren nach einem der Ansprüche 1 bis 3, **dadurch gekennzeichnet, dass** die leichtsiedenden Nebenbestandteile in einem Absorptions-/Desorptions-Cyclus mittels eines hochsiedenden Lösungsmittels abgetrennt werden.

## Claims

1. A process for preparing 1-butene from n-butane, which comprises the steps
A) provision of an n-butane-comprising feed gas stream a;
B) introduction of the n-butane-comprising feed gas stream a into at least one dehydrogenation zone and nonoxidative catalytic dehydrogenation of n-butane to give a product gas stream b comprising n-butane, 1-butene, 2-butene, butadiene, hydrogen, low-boiling secondary constituents and possibly water vapor;
C) removal of hydrogen, the low-boiling secondary constituents and, if appropriate, water vapor to give a C₄ product gas stream c consisting essentially of n-butane, 1-butene, 2-butene and butadiene;
D) separation of the C₄ product gas stream c into a recycle stream d1 consisting essentially of n-butane and a stream d2 comprising 1-butene, 2-butene and butadiene by extractive distillation and recirculation of the recycle stream d1 consisting essentially of n-butane to the dehydrogenation zone;
E) introduction of the stream d2 comprising 1-butene, 2-butene and butadiene into a selective hydrogenation zone and selective hydrogenation of butadiene to 1-butene and/or 2-butene to give a stream e comprising 1-butene and 2-butene;
F) introduction of the stream e comprising 1-butene and 2-butene and a circulating stream g comprising 1-butene and 2-butene into a distillation zone and isolation of a desired product stream f1 consisting essentially of 1-butene to leave a 2-butene-comprising stream f2;
G) introduction of the 2-butene-comprising stream f2, if appropriate after a purge gas stream has been separated off, into an isomerization zone and isomerization of 2-butene to 1-butene so as to give a circulating stream g comprising 1-butene and 2-butene and recirculation, if appropriate after a purge gas stream has been separated off from the circulating gas stream g, of the circulating gas stream g to the distillation zone.

2. The process according to claim 1, wherein the nonoxidative, catalytic dehydrogenation is carried out as an autothermal dehydrogenation.

3. The process according to claim 1 or 2, wherein the extractive distillation is carried out using an N-methylpyrrolidone/water mixture as extractant.

4. The process according to any of claims 1 to 3, wherein the low-boiling secondary constituents are separated off by means of a high-boiling solvent in an absorption/desorption cycle.

## Revendications

1. Procédé de fabrication de 1-butène à partir de n-butane selon les étapes :
A) mise à disposition d'un courant gazeux d'entrée a contenant du n-butane ;
B) introduction du courant gazeux d'entrée a contenant du n-butane dans au moins une zone de déshydrogénation et déshydrogénation catalytique non oxydative du n-butane, un courant gazeux de produits b contenant du n-butane, du 1-butène, du 2-butène, du butadiène, de l'hydrogène, des constituants secondaires de faible point d'ébullition et éventuellement de la vapeur d'eau étant obtenu ;
C) séparation de l'hydrogène, des constituants secondaires de faible point d'ébullition et éventuellement de la vapeur d'eau, un courant gazeux de produits en C₄ c constitué essentiellement de n-butane, de 1-butène, de 2-butène et de butadiène étant obtenu ;
D) séparation du courant gazeux de produits en C₄ c en un courant de recyclage d1 constitué essentiellement de n-butane et un courant d2 contenant du 1-butène, du 2-butène et du butadiène par distillation extractive et recyclage du courant de recyclage d1 constitué essentiellement de n-butane dans la zone de déshydrogénation ;
E) introduction du courant d2 contenant du 1-butène, du 2-butène et du butadiène dans une zone d'hydrogénation sélective et hydrogénation sélective du butadiène en 1-butène et/ou 2-butène, un courante contenant du 1-butène et du 2-butène étant obtenu ;
F) introduction du courant e contenant du 1-butène et du 2-butène et d'un courant circulaire g contenant du 1-butène et du 2-butène dans une zone de distillation et séparation d'un courant de produit de valeur f1 constitué essentiellement de 1-butène, un courant f2 contenant du 2-butène demeurant ;
G) introduction du courant f2 contenant du 2-butène, éventuellement après séparation d'un courant gazeux de purge, dans une zone d'isomérisation et isomérisation du 2-butène en 1-butène, un courant circulaire g contenant du 1-butène et du 2-butène étant obtenu, et recyclage, éventuellement après séparation d'un courant gazeux de purge du courant circulaire g, du courant circulaire g dans la zone de distillation.

2. Procédé selon la revendication 1, **caractérisé en ce que** la déshydrogénation catalytique non oxydative est réalisée sous la forme d'une déshydrogénation autotherme.

3. Procédé selon la revendication 1 ou 2, **caractérisé en ce que** la distillation extractive est réalisée avec un mélange N-méthylpyrrolidone/eau en tant qu'agent d'extraction.

4. Procédé selon l'une quelconque des revendications 1 à 3, **caractérisé en ce que** les constituants secondaires de faible point d'ébullition sont séparés dans un cycle absorption/désorption au moyen d'un solvant de point d'ébullition élevé.
